# EUROPEAN PATENT APPLICATION

(11) **EP 3 533 434 A2**
(43) Date of publication of application: **04.09.2019**
(21) Application number: 18847732.7
(22) Date of filing: 14.08.2018
(51) Int. Cl.: A61K 8/34, A61K 8/9789, A61Q 19/00

(54) **PRESERVATIVE COMPOSITION FOR COSMETICS**

(30) Priority: 22.08.2017 KR 20170105941
(71) Applicant: Kim, Bong Chul, Incheon 21624 (KR)
(72) Inventor: Kim, Bong Chul, Incheon 21624 (KR)
(74) Representative: ABG Intellectual Property Law, S.L.
(86) International application number: PCT/KR2018/009326
(87) International publication number: WO 2019/039788

(57) **Abstract**

Provided is a preservative composition for cosmetics including 1,2-hexanediol, caprylyl glycol, butylene glycol, pentylene glycol, ethylhexylglycerin, an aspalathus linearis extract, a glycyrrhiza glabra (Licorice) root extract, a commiphora myrrha resin extract, a perilla frutescens leaf extract, and a yucca schidigera root extract. The preservative composition for cosmetics of the present invention prevents cosmetics from becoming rancid and is not harmful or toxic, thereby making it possible to produce cosmetics without skin irritation and with excellent moisture retention.

## Description

### TECHNICAL FIELD

The present invention disclosed herein relates to a preservative composition for cosmetics.

### BACKGROUND ART

As an essential ingredient used in cosmetics, a preservative ingredient is required for preventing rancidity of the contents. In this regard, parabens-based chemicals, which have traditionally been used as preservatives for cosmetics and household items, have been frequently mentioned to be harmful for many years. In addition, each country has enacted the Cosmetics Act focusing on strengthening restrictions of cosmetic ingredients which may contain carcinogens or may affect genitalia, and attention concerning the preservatives for cosmetics has been risen.

Accordingly, the necessity for alternative preservatives for cosmetics which are not harmful and toxic is rising, and the industry is striving to develop preservatives for cosmetics which may be naturally safer and may replace parabens. In addition, the development of new products containing preservatives, which do not contain parabens at all, is actively proceeding.

### DISCLOSURE OF THE INVENTION

### TECHNICAL PROBLEM

The present invention provides to produce a preservative composition for cosmetics which are not harmful and toxic.

The present invention also provides a composition having a moisturizing effect as well as a preservative effect.

### TECHNICAL SOLUTION

In accordance with an embodiment of the present invention, a preservative composition is characterized by including 1,2-hexanediol, caprylyl glycol, butylene glycol, pentylene glycol, ethylhexylglycerin, an aspalathus linearis extract, a glycyrrhiza glabra (Licorice) root extract, a commiphora myrrha resin extract, a perilla frutescens leaf extract, and a yucca schidigera root extract.

A purpose of the present invention is to produce a preservative composition for cosmetics which is not harmful or toxic. A composition of the present invention is intended to have a moisturizing effect as well as a preservative effect. and

The commiphora myrrha may have mild nature, have a bitter taste, and be atoxic. The commiphora myrrha may break bound-masses and blood stasis, and make pain stop. The commiphora myrrha may cure when bruised, bones and tendons are injured or broken to have blood stasis and feel pain, wounded by metals, wounded by being whipped, having various malignant furuncles, and having anal fistula. In addition, the commiphora myrrha may soothe toxic swelling, stop sudden bloody discharge, and cure, due to cloudy cornea and conjunctiva (nebula), dizziness, pain and standing out veins in circumference of the cornea.

The *Perilla frutescens* may have warm nature, have a spicy flavor, and be atoxic. The *Perilla frutescens* may cure epigastric swelling and abdominal distension, acute gastroenteritis (vomiting and diarrhea) and beriberi, and may also allow urine and feces come out well. The *Perillafrutescens* may eliminate cold-air in a body and defeat a wind-cold pathogen when you have wind-cold dyspnea (severe asthma due to wind-cold). In addition, the *Perilla frutescens* may make phlegm and energy in a chest come down. Such a perilla frutescens leaf may have effects on anti-inflammatory, tumor suppression, and anti-allergic reaction.

The yucca schidigera root extract is a substance obtained by extracting yucca root with water, and diluent and other food additives may be added for quality preservation. The yucca may be a dark-brown liquid and a flavoring agent with an unusual odor, and may contain saponin glycosides to cause air bubbles when shaken with water. When added to soft drinks, cocktails, etc., the bubbles may be kept for a long time, and the bubbles may be formed at pH 2-10 with stability.

The commiphora myrrha resin extract, perilla frutescens leaf extract, and yucca schidigera root extracts may be each contained in amount of 0.1-0.5 wt% in the total composition. When the content is less than 0.1 wt%, the preservative effect may be insignificant, and when the content exceeds 0.5 wt%, irritation to the skin may be caused.

The aspalathus linearis is a conifer belonging to a leguminous plant, and may have no caffeine and be rich in minerals to be used as tea and good for skin health. In addition, the aspalathus linearis may also protect the skin from free radicals which cause skin aging in the skin exposed to ultraviolet rays, thereby preventing oxidative damage of the skin and helping to manage and relieve skin wrinkles.

The glycyrrhiza glabra (Licorice) may have effects on detoxification, relieving hangovers, anti-inflammation, anti-cancer, curing wound, and metabolism stimulation of skin function.

The aspalathus linearis extract and glycyrrhiza glabra (Licorice) root extracts may be each contained in amount of 0.1-0.5 wt%. When the content is less than 0.1 wt%, the effects of skin moisturizing and anti-aging may be insignificant, and when the content exceeds 0.5 wt%, irritation to the skin may be caused.

In accordance with another embodiment of the present invention, cosmetics having excellent moisturizing and anti-aging effects are characterized by including the preservative composition which has the described composition.

### ADVANTAGEOUS EFFECTS

The preservative composition for cosmetics of the present invention prevents cosmetics from becoming rancid and is not harmful or toxic, thereby making it possible to produce cosmetics without skin irritation.

Furthermore, the composition of the present invention may produce functional cosmetics having not only an excellent preservative effect but also an excellent moisturizing effect.

### BRIEF DESCRIPTION OF THE DRAWINGS

FIGS. 1 to 6 are results and photographs in which bacterial growth of a composition in Table 1 was experimented;

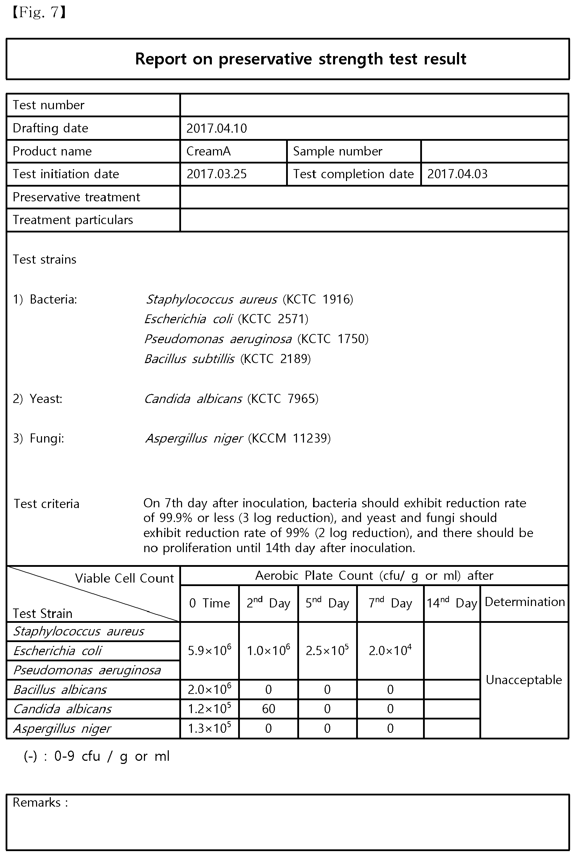

FIG. 7 is a report on preservative strength test result of cream formulation A in Example 2;

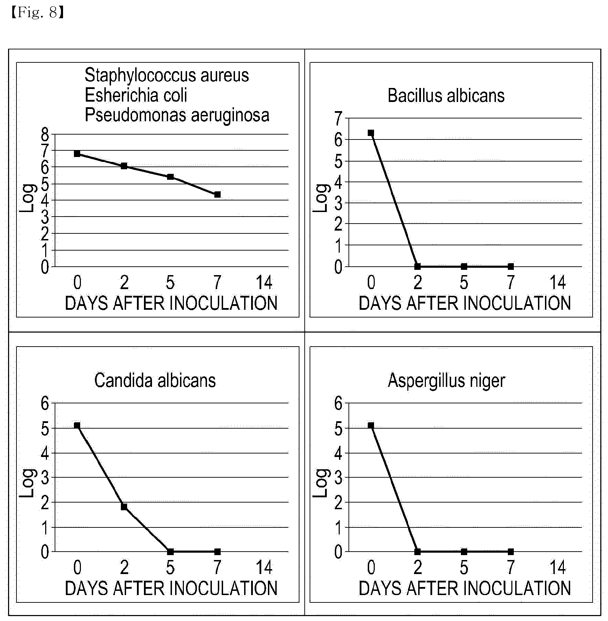

FIG. 8 is a log
   arithmic decrement chart of cream formulation A in Example 2;

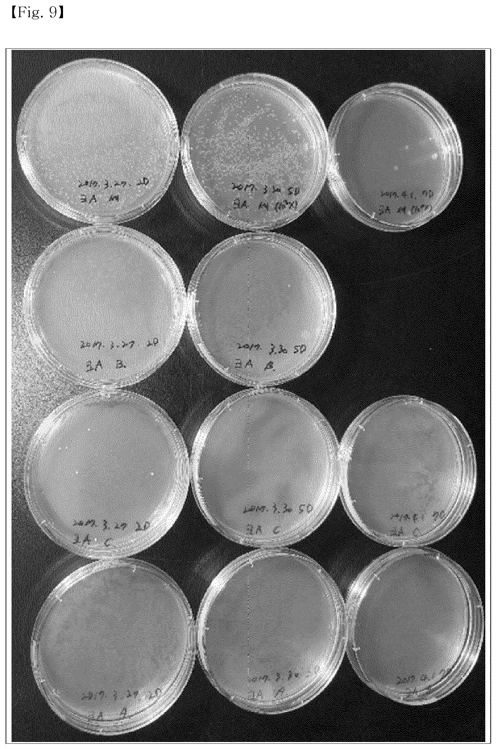

FIG. 9 is a photograph of a bacterial growth test of cream formulation A in Example 2;

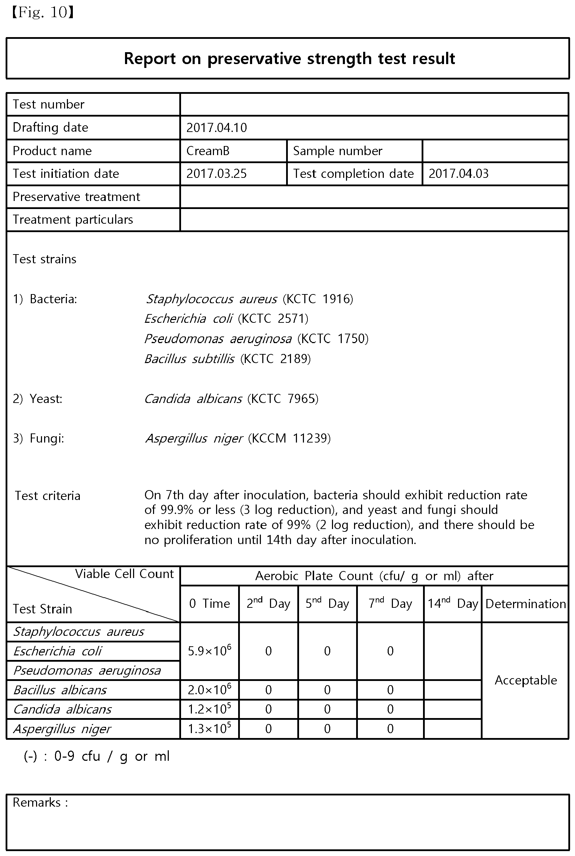

FIG. 10 is a report on preservative strength test result of cream formulation B in Example 2;

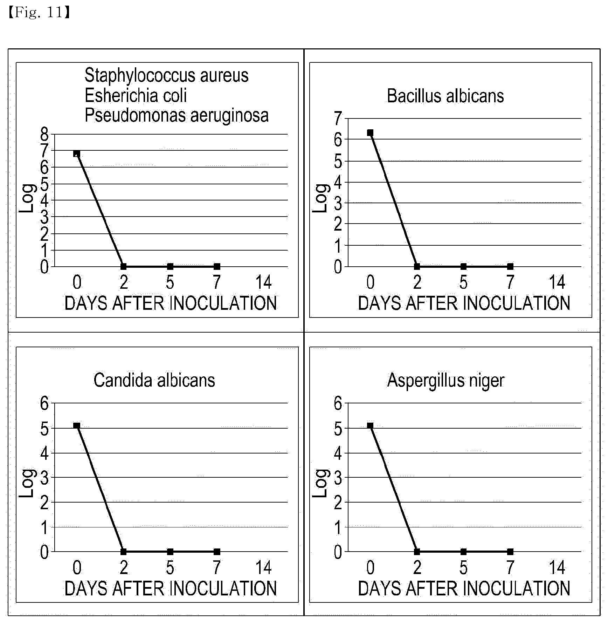

FIG. 11 is a logarithmic decrement chart of cream formulation B in Example 2;

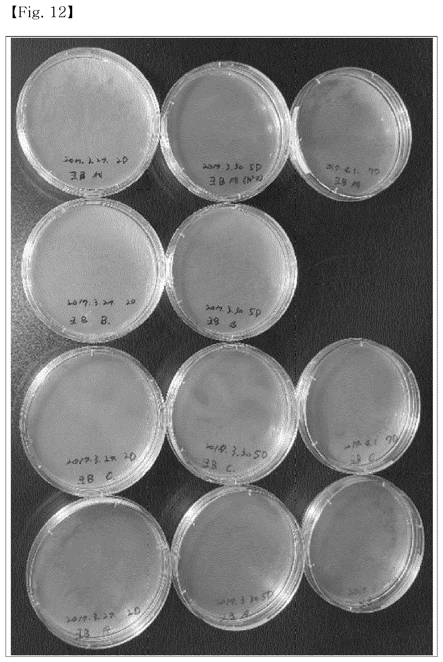

FIG. 12 is a photograph of a bacterial growth test of cream formulation B in Example 2;

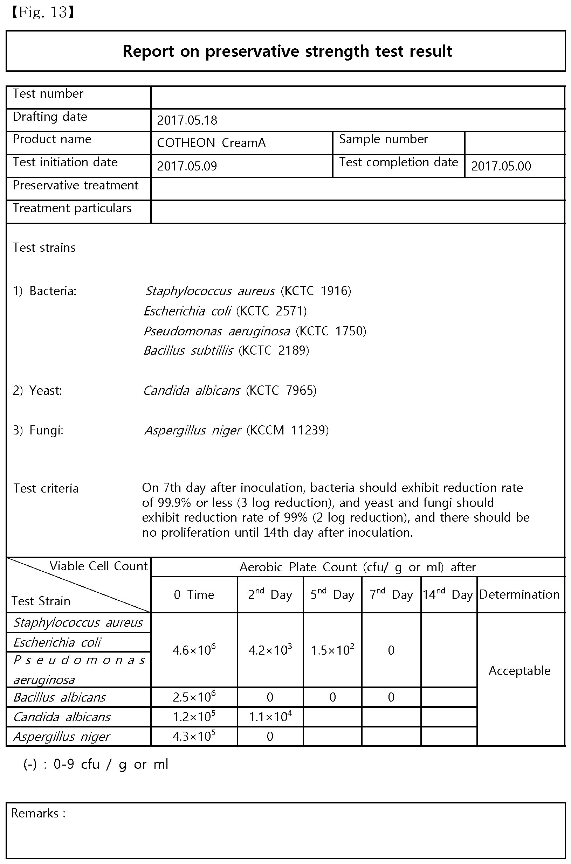

FIG. 13 is a report on preservative strength test result of cream containing a preservative composition in Example 3 of the present invention;

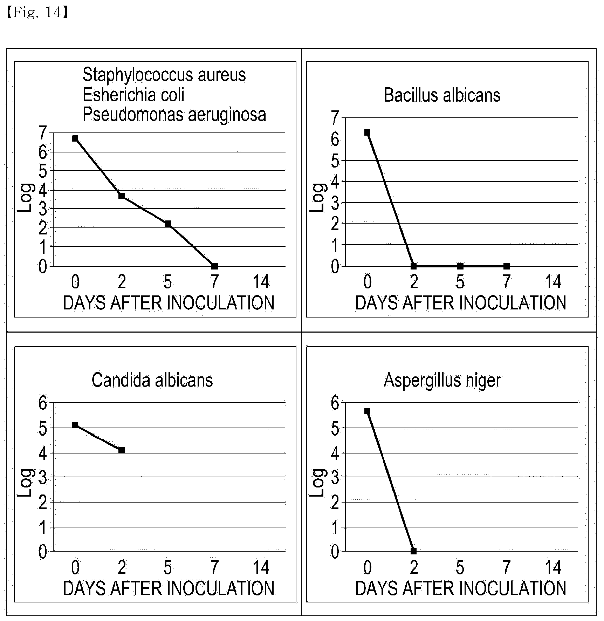

FIG. 14 is a logarithmic decrement chart of cream containing a preservative composition in Example 3 of the present invention; and

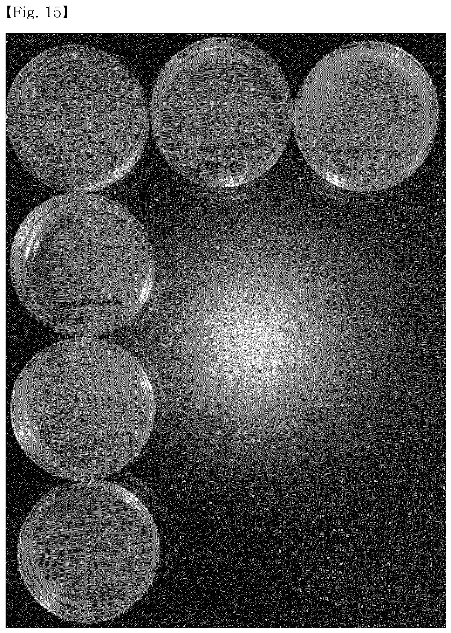

FIG. 15 is a photograph of a bacterial growth test of cream containing a preservative composition in Example 3 of the present invention.

### MODE FOR CARRYING OUT THE INVENTION

Hereinafter, the present invention will be described in more detail with reference to examples.

### Example 1: First experiment

The experiment was performed such that three-type (A, B, and C) preservative compositions as shown in Table 1 were respectively contained in the basic base of Table 2 in an amount of 0.8%.

The reason for adding the aspalathus linearis extract and glycyrrhiza glabra (Licorice) root extracts for the first time was that caprylyl glycol was highly irritated among moisturizers and there was concern of irritation, so that the extracts were each added in a content of 0.5% to relieve irritation.

**[Table 1]**

| A | | B | | C | |
|---|---|---|---|---|---|
| 1,2-hexanediol | 69% | 1,2-hexanediol | 64% | 1,2-hexanediol | 59% |
| Caprylyl glycol | 25% | Caprylyl glycol | 30% | Caprylyl glycol | 35% |
| Butylene glycol | 5% | Butylene glycol | 5% | Butylene glycol | 5% |
| Aspalathus Linearis extract | 0.5% | Aspalathus Linearis extract | 0.5% | Aspalathus Linearis extract | 0.5% |
| Glycyrrhiza Glabra (Licorice) Root extract | 0.5% | Glycyrrhiza Glabra (Licorice) Root extract | 0.5% | Glycyrrhiza Glabra (Licorice) Root extract | 0.5% |

**[Table 2]**

| Raw material name | Content (%) |
|---|---|
| Purified water | to 100 |
| Glycerin | 1.00 |
| Sorbitol | 3.00 |
| Xanthan gum | 0.15 |
| Hydroxyethyl cellulose | 0.15 |
| Cetearyl alcohol | 4 |
| Sorbitan stearate | 0.7 |
| Polysorbate 60 | 1 |
| Mineral oil | 6 |
| Canola oil | 5 |
| Dimethicone | 0.3 |
| Preservative raw material (A, B, C) | 0.8 |

### 1. Experimental conditions

### 2. Test strains

1) Bacteria: Staphylococcus aureus (KCTC 1916)
   Escherichia coli (KCTC 2571)
   Pseudomonas aeruginosa (KCTC 1750)
   Bacillus subtillis (KCTC 2189)
2) Yeast: Candida albicans (KCTC 7965)
3) Fungi: Aspergillus niger (KCCM 11239)

### 3. Test criteria

On 7th day after inoculation, bacteria should exhibit reduction rate of 99.9% or less (3 log reduction), and yeast and fungi should exhibit reduction rate of 99% (2 log reduction), and there should be no proliferation until 14th day after inoculation.

### 4. Experimental results

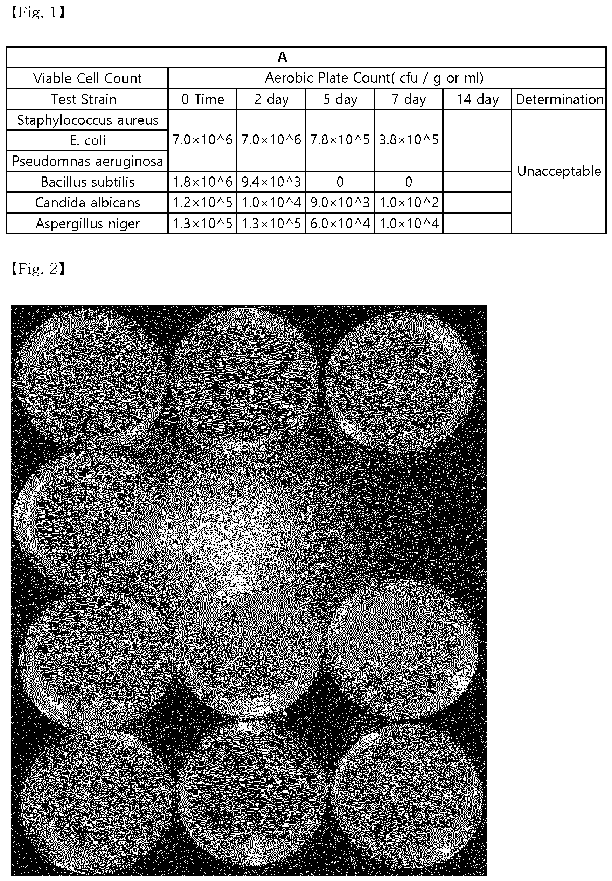

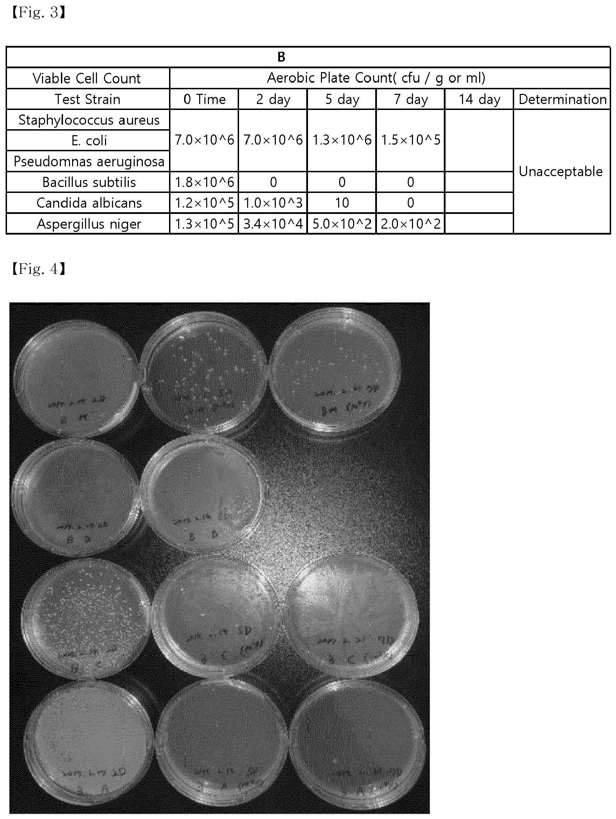

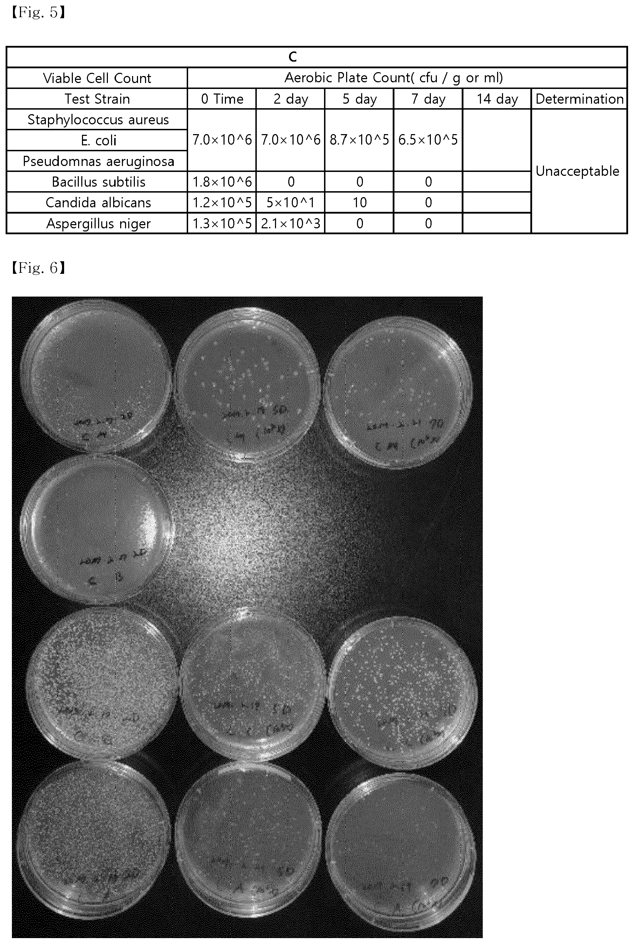

Results of the experiment are shown in FIGS. 1 to 6, and summary of each group is shown in Table 4 below.

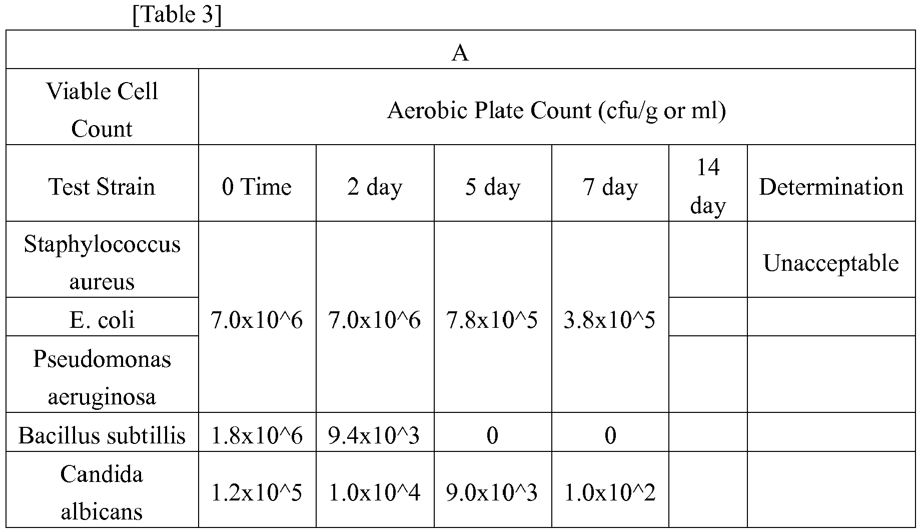

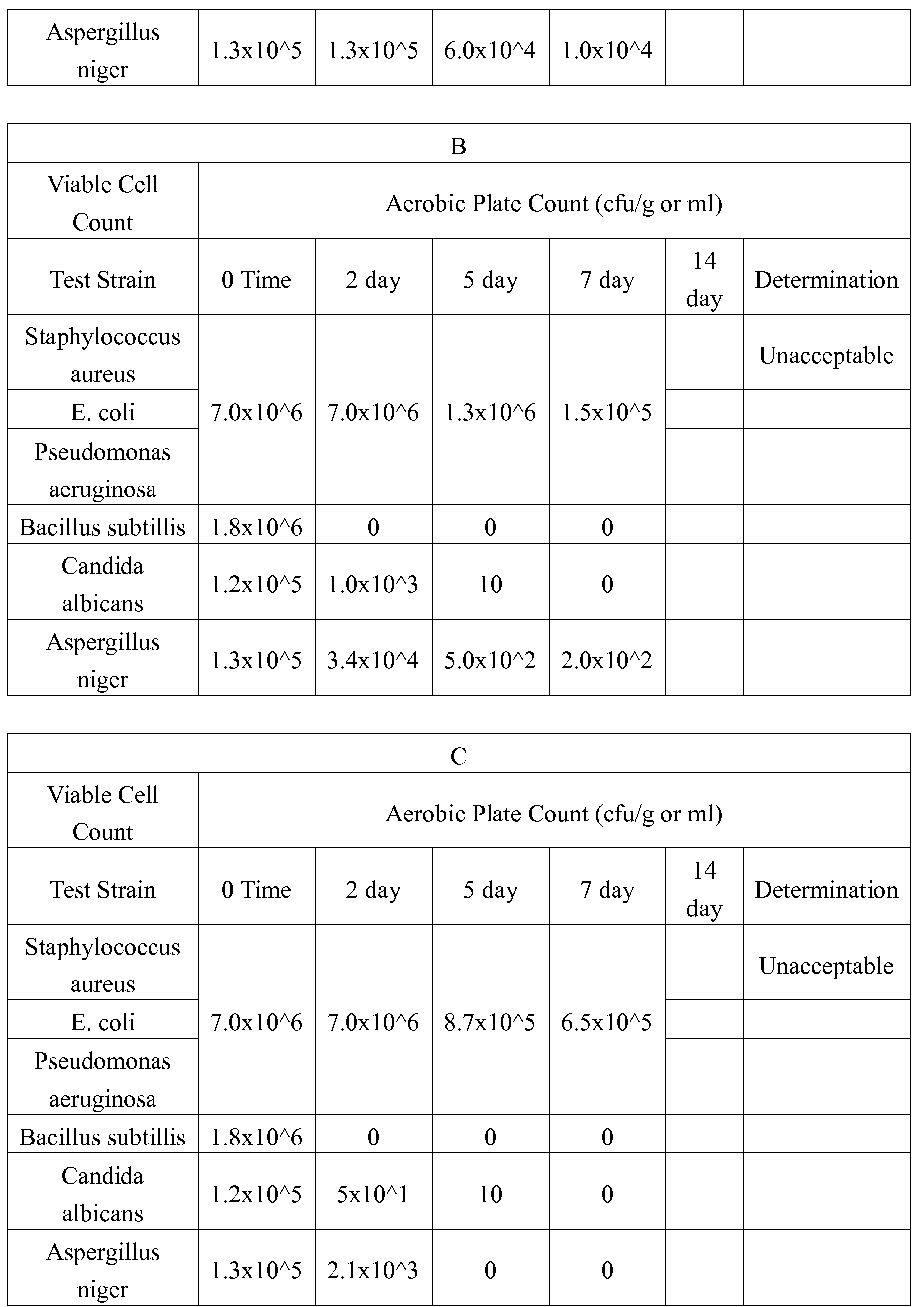

In the experimental results as shown in FIGS. 1 to 6, the preservative composition including 1,2-hexanediol, caprylyl glycol, butylene glycol, aspalathus linearis extract, and a glycyrrhiza glabra (Licorice) root extract did not meet the criteria for the reduction rate and proliferation effect of the test strains.

### Example 2: Second experiment

Based on the derivation in the first experiment, the content of each ingredient was modified and new ingredients (pentylene glycol and ethylhexylglycerin) were added. The added amount was 0.8%, respectively, the same as before.

commiphora myrrha resin extract, perilla frutescens leaf extract, and yucca schidigera root extract were added to B, and the basic base was the same as in the first experiment.

**[Table 4]**

| A | | B | |
|---|---|---|---|
| 1,2-hexanediol | 57% | 1,2-hexanediol | 56.1% |
| Caprylyl glycol | 33% | Caprylyl glycol | 33% |
| Butylene glycol | 5% | Butylene glycol | 5% |
| Ethylhexylglycerin | 3% | Ethylhexylglycerin | 3% |
| Pentylene glycol | 1% | Pentylene glycol | 1% |
| | | Aspalathus Linearis extract | 0.5% |
| Aspalathus Linearis extract | 0.5% | Glycyrrhiza Glabra (Licorice) Root extract | 0.5% |
| | | Commiphora Myrrha Resin extract | 0.3% |
| Glycyrrhiza Glabra (Licorice) Root extract | 0.5% | Perilla Frutescens Leaf extract | 0.3% |
| | | Yucca Schidigera Root extract | 0.3% |

**[Table 5]**

| Raw material name | Content (%) |
|---|---|
| Purified water | to 100 |
| Glycerin | 1.00 |
| Sorbitol | 3.00 |
| Xanthan gum | 0.15 |
| Hydroxyethyl cellulose | 0.15 |
| Cetearyl alcohol | 4 |
| Sorbitan stearate | 0.7 |
| Polysorbate 60 | 1 |
| Mineral oil | 6 |
| Canola oil | 5 |
| Dimethicone | 0.3 |
| Preservative raw material (A, B) | 0.8 |

### 1. Experimental conditions

### 2. Test strains

1) Bacteria: Staphylococcus aureus (KCTC 1916)
   Escherichia coli (KCTC 2571)
   Pseudomonas aeruginosa (KCTC 1750)
   Bacillus subtillis (KCTC 2189)
2) Yeast: Candida albicans (KCTC 7965)
3) Fungi: Aspergillus niger (KCCM 11239)

### 3.Test criteria

On 7th day after inoculation, bacteria should exhibit reduction rate of 99.9% or less (3 log reduction), and yeast and fungi should exhibit reduction rate of 99% (2 log reduction), and there should be no proliferation until 14th day after inoculation.

### 4. Experimental results

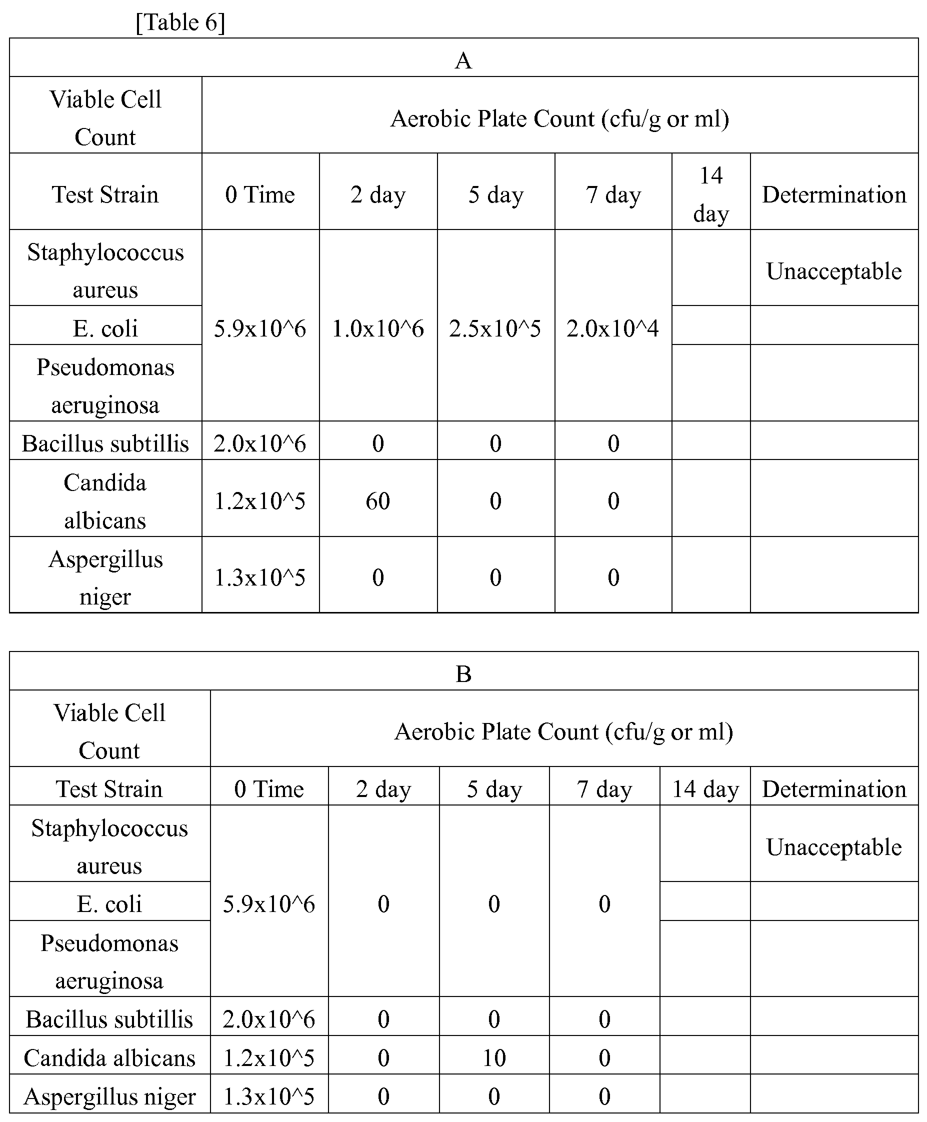

The content and ingredients of the remainders were the same except for the three extracts added in the second experiment, but numerical differences were shown. In conclusion, it may be considered that commiphora myrrha resin extract, perilla frutescens leaf extract, and yucca schidigera root extract are helpful for the products.

### Example 3: Third experiment

The experiment was performed such that the content of the added three extracts was reduced from 0.3% to 0.1%.

**Table 7]**

| A | |
|---|---|
| 1,2-hexanediol | 56.7% |
| Caprylyl glycol | 33% |
| Butylene glycol | 5% |
| Pentylene glycol | 3% |
| Ethylhexylglycerin | 1% |
| Aspalathus Linearis extract | 0.5% |
| Glycyrrhiza Glabra (Licorice) Root extract | 0.5% |
| Commiphora Myrrha Resin extract | 0.1% |
| Perilla Frutescens Leaf extract | 0.1% |
| Yucca Schidigera Root extract | 0.1% |

**[Table 8]**

| Raw material name | Content (%) |
|---|---|
| Purified water | to 100 |
| Glycerin | 1.00 |
| Sorbitol | 3.00 |
| Xanthan gum | 0.15 |
| Hydroxyethyl cellulose | 0.15 |
| Cetearyl alcohol | 4 |
| Sorbitan stearate | 0.7 |
| Polysorbate 60 | 1 |
| Mineral oil | 6 |
| Canola oil | 5 |
| Dimethicone | 0.3 |
| Preservative raw material (A) | 0.8 |

### 1. Experimental conditions

### 2. Test strains

1) Bacteria: Staphylococcus aureus (KCTC 1916)
   Escherichia coli (KCTC 2571)
   Pseudomonas aeruginosa (KCTC 1750)
   Bacillus subtillis (KCTC 2189)
2) Yeast: Candida albicans (KCTC 7965)
3) Fungi: Aspergillus niger (KCCM 11239)

### 3. Test criteria

On 7th day after inoculation, bacteria should exhibit reduction rate of 99.9% or less (3 log reduction), and yeast and fungi should exhibit reduction rate of 99% (2 log reduction), and there should be no proliferation until 14th day after inoculation.

### 4. Experimental results

**[Table 9]**

| A | | | | | | |
|---|---|---|---|---|---|---|
| Viable Cell Count | Aerobic Plate Count (cfu/g or ml) | | | | | |
| Test Strain | 0 Time | 2 day | 5 day | 7 day | 14 day | Determination |
| Staphylococcus aureus | 4.6x10^6 | 4.2x10^3 | 1.5x10^2 | 0 | | Acceptable |
| E. coli | | | | | | |
| Pseudomonas aeruginosa | | | | | | |
| Bacillus subtillis | 2.5x10^6 | 0 | 0 | 0 | | |
| Candida albicans | 1.2x10^5 | 1.1x10^4 | 0 | 0 | | |
| Aspergillus niger | 4.3x10^5 | 0 | 0 | 0 | | |

* The preservative effect was the same even when the content of the commiphora myrrha resin extract, perilla frutescens leaf extract, and yucca schidigera root extracts was reduced to 0.1 wt%, respectively.

### Example 4: Sensory test

* Cosmetic including the preservative compositions in Examples 2 and 3 and the base in Table 8 was prepared, and 30 women in their 20s-50s were selected and used for 6 months to test whether the skin was irritated.

In every morning and evening, 1g was applied to the face and absorbed, and the skin irritation and moisture retention were measured and the satisfaction was examined (5. No skin irritation, Excellent moisture retention, 4. Slight skin irritation, Good moisture retention, 3. Normal skin irritation, Normal moisture retention, 2. A lot of skin irritation, little moisture retention, 1. Very much skin irritation, no moisture retention).

Almost all 30 subjects answered that they did not feel skin irritation, and it was confirmed that the skin was not dry and kept moisturized (skin irritation; average 4.8, moisture retention: average 4.7). As a result of the evaluation, the composition of the present invention has an excellent preservative effect on preventing bacterial growth in cosmetics without skin irritation, has an excellent effect on improving the skin moisture retention, and provides the users with satisfaction.

## Claims

1. A natural preservative composition for cosmetics having skin irritation relief and moisture retention, the composition comprising 1,2-hexanediol, caprylyl glycol, butylene glycol, pentylene glycol, ethylhexylglycerin, aspalathus linearis extract, a glycyrrhiza glabra (Licorice) root extract, a commiphora myrrha resin extract, a perilla frutescens leaf extract, and yucca schidigera root extract.

2. The natural preservative composition of claim 1, wherein the commiphora myrrha resin extract, perilla frutescens leaf extract, and yucca schidigera root extracts are contained in amount of 0.1-0.3 wt%.

3. The natural preservative composition of claim 1, wherein the aspalathus linearis extract and glycyrrhiza glabra (Licorice) root extracts are contained in amount of 0.1-0.5 wt%.

4. A cosmetic having skin irritation relief and moisture retention, the cosmetic comprising the composition of any one of claims 1 to 3.
